(19) Europäisches Patentamt
European Patent Office
Office européen des brevets

(11) EP 2 719 416 B1

(12) EUROPEAN PATENT SPECIFICATION

(45) Date of publication and mention
of the grant of the patent:
**14.06.2017 Bulletin 2017/24**

(21) Application number: **11871255.3**

(22) Date of filing: **19.08.2011**

(51) Int Cl.:
**A61M 15/06** (2006.01)    **A61M 11/06** (2006.01)

(86) International application number:
**PCT/JP2011/068783**

(87) International publication number:
**WO 2013/027249 (28.02.2013 Gazette 2013/09)**

(54) **AEROSOL INHALER**

AEROSOL INHALATOR

AEROSOL INHALATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.04.2014 Bulletin 2014/16**

(73) Proprietor: **JAPAN TOBACCO INC.**
**Minato-ku**
**Tokyo 105-8422 (JP)**

(72) Inventors:
• **YAMADA, Manabu**
**Tokyo 130-8603 (JP)**

• **SASAKI, Hiroshi**
**Tokyo 130-8603 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(56) References cited:
**EP-A1- 0 845 220      EP-A1- 0 893 071**
**EP-A1- 2 213 321      WO-A1-95/01137**
**CH-B1- 698 603        JP-A- 2009 131 367**
**JP-A- 2009 131 367    JP-B2- 3 325 028**
**US-A- 5 666 977**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to an aerosol inhalator capable of generating an aerosol as a user inhales, to supply the user with the generated aerosol.

### Citation List

### Patent Literature

Patent Document 1: PCT International Publication No. WO 2008/105918 A1
Patent Document 2: PCT International Application-Japanese Translation No. JP 2006-524494 A
Patent Document 3: PCT International Application-Japanese Domestic Re-publication No. WO 97/48293
Patent Document 4: Unexamined Japanese Patent Publication No. JP H11(1999)-89551
Patent Document 5: PCT International Application WO 95/01137 A1
Patent Document 6: European Patent Application EP 0 845 220 A1

### Background Art

This type of aerosol inhalator is disclosed, for example, in Patent Documents 1 to 4 identified below.

The aerosol inhalator disclosed in Patent Document 1 includes a suction pipe provided with a mouthpiece, a solution supply source incorporated into the suction pipe and storing a solution to be aerosolized, a dispenser capable of supplying a fixed amount of the solution at a time from the solution supply source to a dispensing position within the suction pipe, and an electric heater for heating and thereby atomizing the solution supplied to the dispensing position, to generate an aerosol inside the suction pipe.

The aerosol inhalator disclosed in Patent Document 2 includes an electric heater and a high-frequency generator, in order to aerosolize a liquid fed by a pump.

The aerosol inhalator disclosed in Patent Document 3 includes an ink jet unit for aerosolizing a liquid.

The aerosol inhalator disclosed in Patent Document 4 includes a liquid supply path utilizing capillarity, and an electric heater arranged at the outlet of the liquid supply path.

Patent Document 5 shows a dispenser with a reservoir of an active substance and a droplet ejection device, which is controlled to issue a predetermined number of discrete droplets of substance from ejection orifices upon actuation.

Patent Document 6 describes a flavor generation article with a casing constituted by first and second portions that are detachably connected to each other. A gas flow path is formed in the first portion of a casing to extend from an air intake port to reach a suction port. A first portion incorporates a material container of a liquid material containing a flavor substance. A discharge port of a material container is arranged in the gas flow path and a ceramic heater is disposed to opposite. Liquid material is supplied from the discharge port and to the ceramic heater and is heated, so that it is gasified in the gas flow path. The second portion of a casing has a control unit and a power supply.

### Summary of Invention

### Technical Problem

The aerosol inhalator of Patent Document 1 requires that the user manually operate the dispenser before inhaling through the mouthpiece, or that the dispenser automatically operate simultaneously with the user's inhalation. The use of the dispenser directly leads to increase in the size of the aerosol inhalator, and also the need for manual operation of the dispenser is a hindrance to the user's easy inhalation of the aerosol.

Automatic operation of the dispenser enables the user to inhale the aerosol with ease, but in this case, the dispenser not only requires a complicated structure but consumes electrical energy for the automatic operation. Consequently, a high-capacity power supply is indispensable for the dispenser and the electric heater, resulting in further increase in the size of the aerosol inhalator.

In the case of the aerosol inhalators of Patent Documents 2 and 3, it is difficult to reduce the sizes of the aerosol inhalators because of their complicated structures, like the aerosol inhalator of Patent Document 1. The aerosol inhalator of Patent Document 4, on the other hand, has a simple structure, compared with the aerosol inhalators of Patent Documents 1 to 3. However, like the aerosol inhalators of Patent Documents 1 to 3, the liquid is aerosolized not by causing the liquid to collide directly with the electric heater, and thus reliable aerosolization is not guaranteed.

An object of the present invention is to provide a small-sized aerosol inhalator which enables a user to inhale an aerosol with ease and also guarantees reliable aerosolization of liquid.

**Solution to Problem**

The above object is achieved by an aerosol inhalator of the present invention, which comprises:

a suction path connecting an atmosphere-exposed opening and a mouthpiece to each other and permitting air to flow from the atmosphere-exposed opening toward the mouthpiece;
a solution supply device configured to supply a solution from which an aerosol is to be generated, the solution supply device including
a solution supply source storing the solution, and
a capillary tube connected to the solution supply source and having a discharge end located in the suction path and opening in a direction toward the mouthpiece, the capillary tube guiding the solution from the solution supply source to the discharge end and, when the flow of air is produced within the suction path, allowing the solution to be discharged from the discharge end; and
a heater device configured to receive the solution discharged from the discharge end of the capillary tube and atomize the received solution by heating, the heater device including
a power supply, and
an electric heater arranged immediately downstream of the discharge end and facing the discharge end while permitting the flow of air, the heater being configured to generate heat when applied with a voltage from the power supply, wherein the heater extends in a direction perpendicular to an axis of said suction path and traverses said suction path; wherein the discharge end of the capillary tube is arranged in a position such that the discharge end is hidden by the heater when an interior of said suction path is viewed from the mouthpiece side, wherein a distance is secured between the discharge end of the capillary tube and the heater, and the distance is adapted so that a liquid droplet of the solution is formed at the discharge end by surface tension of the solution and the formed liquid droplet is directly received by a surface of the electric heater.

With the above aerosol inhalator, when the user inhales through the mouthpiece, the solution is discharged from the discharge end of the capillary tube. The discharged solution is received on the outer surface of the heater and at the same time is atomized in its entirety by heat generated by the heater, so that an aerosol is generated inside the suction path. The user can therefore inhale the aerosol through the mouthpiece.

Preferably, the capillary tube extends coaxially with the suction path.

**Advantageous Effects of Invention**

In the aerosol inhalator of the present invention, the solution is discharged from the discharge end of the capillary tube in conjunction with the user's inhalation, and the discharged solution is received on the outer surface of the heater, so that a total amount of the discharged solution can be atomized on the outer surface of the heater, generating an aerosol inside the suction path. Accordingly, the user can easily and effectively inhale the aerosol.

Details and other advantages of the aerosol inhalator of the present invention will become apparent from the following description when taken in conjunction with the accompanying drawings.

**Brief Description of Drawings**

FIG. 1 is a schematic longitudinal sectional view of an aerosol inhalator according to one embodiment of the present invention.
FIG. 2 illustrates a specific example of a liquid tank appearing in FIG. 1.
FIG. 3 is an enlarged sectional view of a heater appearing in FIG. 1.
FIG. 4 illustrates the heater in FIG. 1 along with a power feed circuit.
FIG. 5 schematically illustrates part of the aerosol inhalator in a state before an aerosol is generated.
FIG. 6 schematically illustrates part of the aerosol inhalator in a state in which an aerosol is being generated, as viewed in a longitudinal section of an inner tube taken along a longitudinal section of the heater.
FIG. 7 schematically illustrates part of the aerosol inhalator in a state in which an aerosol is being generated, as viewed in a longitudinal section of the inner tube taken along a cross section of the heater.

FIG. 8 illustrates a malfunction of the aerosol inhalator caused in cases where the distance between a capillary tube and the heater is too long.

FIG. 9 illustrates a malfunction of the aerosol inhalator caused in cases where the distance between the capillary tube and the heater is too short.

FIG. 10 schematically illustrates a heating test apparatus for determining an optimum heater.

FIG. 11 is a graph showing measurement results obtained by the heating test apparatus.

FIG. 12 illustrates a modification of a sheath element.

## Description of Embodiments

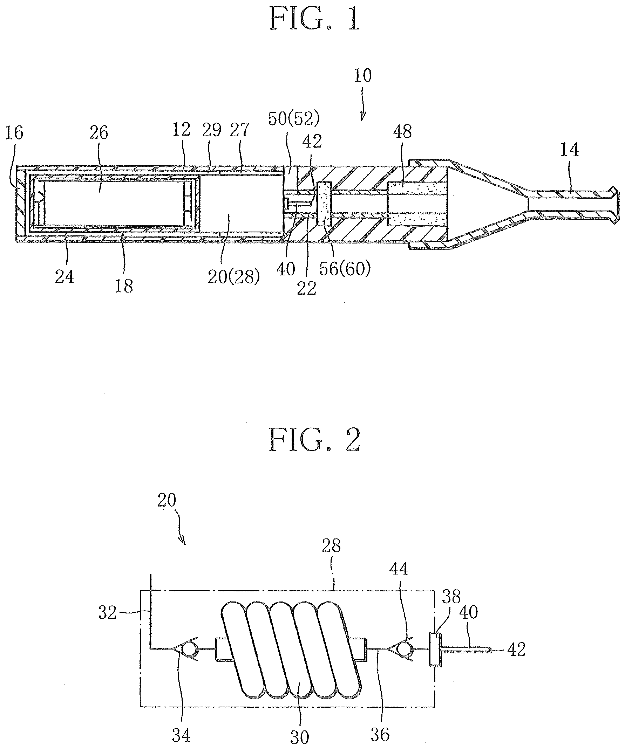

As illustrated in FIG. 1, an aerosol inhalator 10 according to one embodiment includes a cylindrical outer tube 12 opening at both ends, and a mouthpiece 14 detachably connected to a proximal end of the outer tube 12. The outer tube 12 and the mouthpiece 14 are each made of heat-resistant synthetic resin. The outer tube 12 has a lid 16 located at its distal end, and the lid 16 is detachable from the outer tube 12.

The outer tube 12 contains a power supply unit 18 as a source of electricity, a liquid tank 20 as a solution supply source, and an inner tube 22. The power supply unit 18, the liquid tank 20 and the inner tube 22 are arranged sequentially in the mentioned order from the lid side coaxially with the outer tube 12. The inner tube 22 communicates with the mouthpiece 14.

The power supply unit 18 and the liquid tank 20 are each replaceable, and with the lid 16 detached from the outer tube 12, the power supply unit 18 and the liquid tank 20 are replaced with new ones.

The power supply unit 18 includes a cell holder 24 and a commercially available battery cell, for example, an AA size cell 26, held by the cell holder 24. The cell 26 has a nominal voltage of 1.5 V and is arranged coaxially with the outer tube 12.

The liquid tank 20 is illustrated in detail in FIG. 2.

The liquid tank 20 includes a cylindrical tank casing 28. The tank casing 28 has a plurality of ribs formed on an outer peripheral surface thereof. The ribs are spaced from each other in a circumferential direction of the tank casing 28 and extend in an axial direction of the tank casing 28 except an end portion of the casing 28 close to the power supply unit 18.

The ribs serve to form a plurality of axial passages 27 (see FIG. 1) between the outer surface of the tank casing 28 and an inner surface of the outer tube 12, and also serve to secure an annular chamber 29 (see FIG. 1) between the aforementioned end portion of the tank casing 28 and the inner surface of the outer tube 12. The annular chamber 29 is connected to the axial passages 27.

A tube coil 30 is contained in the tank casing 28. The tube coil 30 extends in the axial direction of the outer tube 12 and has opposite open ends. An inlet conduit 32 extends from one end of the tube coil 30 to the outer surface of the tank casing 28 and opens on the outer surface of the tank casing 28 to be connected to the annular chamber 29. A check valve 34 is arranged in the inlet conduit 32 and opens only in one direction toward the one end of the tube coil 30.

An outlet conduit 36 extends from the other end of the tube coil 30 and is connected to a capillary tube 40 through a joint 38. The capillary tube 40 projects from the tank casing 28 into the aforementioned inner tube 22 and is located coaxially with the inner tube 22. The projecting end of the capillary tube 40 forms a discharge end 42, which opens in a direction toward the mouthpiece 14. Another check valve 44 is arranged in the outlet conduit 36 and opens only in one direction toward the capillary tube 40.

An internal flow channel (inlet conduit 32, tube coil 30 and outlet conduit 36) of the liquid tank 20 and the capillary tube 40 are filled with a solution to be aerosolized, and the solution reaches the discharge end 42 of the capillary tube 40. The solution may contain, for example, propylene glycol, glycerin or the like.

As is clear from FIG. 1, the inner tube 22 extends from the liquid tank 20 toward the mouthpiece 14 and is connected to an absorbent sleeve 48. The absorbent sleeve 48 is located in alignment with the inner tube 22 and has an inner diameter identical with that of the inner tube 22. A portion of the outer tube 12 surrounding the inner tube 22 and the absorbent sleeve 48 has a larger thickness than a portion of the outer tube 12 surrounding the power supply unit 18 and the liquid tank 20.

Specifically, the inner tube 22 is made of stainless steel or ceramic, for example. On the other hand, the absorbent sleeve 48 is, for example, a paper tube or hollow tubular paper filter capable of absorbing the solution. The absorbent sleeve 48 has a volume sufficient to retain a required absorption amount of the solution.

As illustrated in FIG. 1, the outer tube 12 has a plurality of atmospheric ports 50 formed therein. The atmospheric ports 50 adjoin the liquid tank 20, for example, and are spaced from each other in the circumferential direction of the outer tube 12. Each of the atmospheric ports 50 extends from the outer peripheral surface of the outer tube 12 and penetrates through the inner tube 22. Thus, the atmospheric ports 50 provide atmosphere-exposed openings 52 that open on the outer peripheral surface of the outer tube 12, and are connected to the annular chamber 29 through the axial passages 27.

Accordingly, the atmospheric ports 50 and the inner tube 22 form a suction path connecting the atmosphere-exposed openings 52 to the mouthpiece 14. Also, the atmospheric ports 50 serve to keep the interior of the annular chamber 29 at atmospheric pressure, and as a consequence, the solution in the liquid tank 20 remains in a state such that the solution is always acted upon by the atmospheric pressure through the open end of the inlet conduit 32.

When the user inhales the air in the inner tube 22 through the mouthpiece 14, a negative pressure is created in the inner tube 22, so that ambient air is introduced into the inner tube 22 through the atmospheric ports 50. Such introduction of the ambient air produces, within the suction path, a flow of air toward the mouthpiece 14.

The negative pressure created in the inner tube 22 causes the solution to be discharged from the discharge end 42 of the capillary tube 40 into the suction path, namely, into the inner tube 22, and the amount of the solution discharged is determined by the intensity of the negative pressure. On the other hand, the capillary tube 40 is replenished with the solution from the liquid tank 20 in an amount corresponding to the discharge amount. Since the solution in the liquid tank 20 is always applied with the atmospheric pressure as stated above, the solution in the internal flow channel of the liquid tank 20 moves toward the capillary tube 40 accompanying the replenishment of the solution.

A cylindrical heater 56 is arranged in the inner tube 22. The heater 56 is located immediately downstream of the discharge end 42 of the capillary tube 40, as viewed in the direction of the flow of air produced in the suction path.

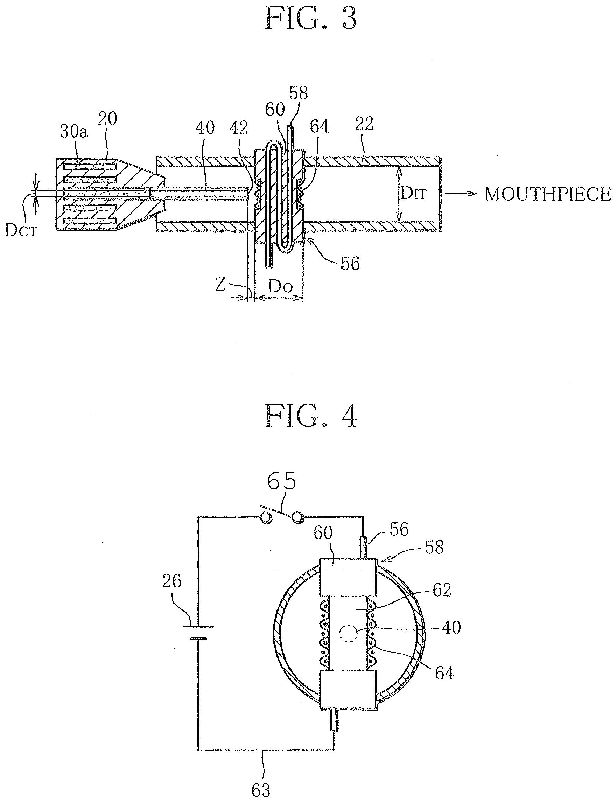

Provided that as shown in FIG. 3, the inner diameters of the inner tube 22 and capillary tube 40 are $D_{IT}$ and $D_{CT}$, respectively, the outer diameter $D_O$ of the heater 56 is smaller than the inner diameter $D_{IT}$ of the inner tube 22 and at the same time is larger than the inner diameter $D_{CT}$ or outer diameter of the capillary tube 40.

That is, the outer diameter $D_O$ satisfies the following relationship:

$$D_{IT} > D_O > D_{CT} \qquad ---(1)$$

The heater 56 penetrates through the inner tube 22 in a diametrical direction of the tube 22 and has an axis intersecting perpendicularly with the axis of the inner tube 22. The heater 56 is supported at both ends by the outer tube 12.

Considering that the capillary tube 40 is located coaxially with the inner tube 22 as stated above, the discharge end 42 of the capillary tube 40 is hidden by the heater 56 when the heater 56 is viewed from the downstream end of the inner tube 22. In other words, the cross section of the discharge end 42 can be totally projected onto the outer surface of the heater 56.

Further, when the solution is discharged from the discharge end 42 in the aforementioned manner, the discharged solution forms a liquid droplet at the discharge end 42, and a maximum diameter of the liquid droplet is determined by the inner diameter $D_{CT}$ of the capillary tube 40. Provided the maximum diameter of the liquid droplet is $D_{MAX}$, a gap Z between the discharge end 42 and the heater 56 fulfills the following relationship:

$$D_{MAX} > Z > D_{CT} \qquad ---(2)$$

Thus, when the solution is discharged from the discharge end 42, the discharged solution is received on the outer surface of the heater 56 without fail.

Table 1 below shows the relationship observed where the solution is propylene glycol (PG; density: 1.036 g/mm$^2$), among the discharge amount and volume of the solution discharged in the form of a liquid droplet and the diameter of the liquid droplet with respect to the inner diameter $D_{CT}$ of the capillary tube 40 and the flow rate of intake air flowing through the inner tube 22.

[Table 1]

| Solution | Capillary tube | | Intake air flow rate | Discharge amount (mg) | Discharge volume (mm$^3$) | Diameter (mm) |
|---|---|---|---|---|---|---|
| | $D_{CT}$ (mm) | Cross-sectional flow area (mm) | | | | |
| PG | 0.36 | 0.1 | 35 ml/2 sec | 2.58 | 2.49 | 0.84 |
| | | | 55 ml/2 sec | 3 | 2.90 | 0.88 |
| | 0.5 | 0.2 | 35 ml/2 sec | 5.5 | 5.31 | 1.08 |
| | | | 55 ml/2 sec | 11 | 10.62 | 1.36 |

The liquid tank 20 illustrated in FIG. 3 has a structure different from that of the liquid tank already explained above. Specifically, the liquid tank 20 in FIG. 3 has an internal flow channel 30a extending in a zigzag, in place of the coil tube 30. This means that the coil tube 30 is not indispensable to the liquid tank 20.

The structure of the heater 56 will now be described in detail.

The heater 56 includes, for example, a Nichrome wire 58 as a resistance heating element, and a cylindrical sheath element 60 enclosing the Nichrome wire 58. In this embodiment, as is clear from FIG. 3, the Nichrome wire 58 axially penetrates through the sheath element 60 three times and has two ends projecting from the respective opposite ends of the sheath element 60.

As illustrated in FIG. 4, the Nichrome wire 58 is connected in series with the aforementioned cell 26 via a power feed circuit 63, and the power feed circuit 63 has a switch 65. Although not illustrated in FIG. 1, the power feed circuit 63 and the switch 65 are arranged on the inner surface of the outer tube 12, and the outer tube 12 is provided, on its outer surface, with a push button (not shown) for operating the switch 65.

The sheath element 60 is made of a ceramic such as alumina or silicon nitride, and constitutes the outer surface of the heater 56. Further, as is clear from FIG. 4, an annular groove 62, for example, is formed in part of the outer surface of the sheath element 60, and a ringshaped heat-resistant net 64, which serves as a wetting enhancement element, is preferably fitted around the annular groove 62. The net 64 directly faces the discharge end 42 of the capillary tube 40, and the aforementioned gap Z is secured between the discharge end 42 and the net 64.

The sheath element 60 not only protects the Nichrome wire 58 but thermally connects the Nichrome wire 58 and the net 64. Specifically, where the cell 26 is in a usable state and the Nichrome wire 58 is applied with a voltage of 1 to 1.5 V, the sheath element 60 performs the function of quickly transferring heat generated by the Nichrome wire 58 to the outer surface of the heater 56 and keeping the heating temperature of the outer surface of the heater 56 within a temperature range required to atomize the solution. That is, the Nichrome wire 58 and the sheath element 60 constitute an internal structure whereby the heating temperature of the outer surface of the heater 56 is kept within the required temperature range, and to this end, the sheath element 60 has a predetermined thickness and volume.

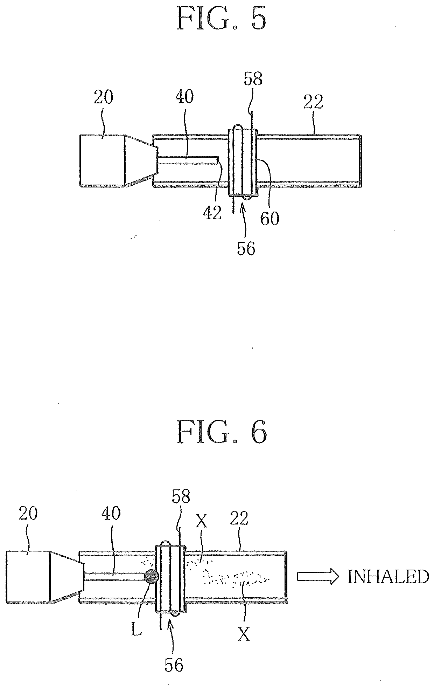

Referring now to FIGS. 5 to 9, the principle of operation of the aerosol inhalator according to the embodiment will be explained. In FIGS. 5 to 9, the net 64 of the heater 56 is not illustrated.

FIG. 5 illustrates a state in which the aerosol inhalator is ready for use with the switch 64 of the power feed circuit 63 turned on. The heating temperature of the outer surface of the heater 56 is quickly raised and kept within the required temperature range, and since the relationship indicated by the aforementioned expression (2) is fulfilled, the solution in the capillary tube 40 is not atomized by the radiant heat from the heater 56. That is, no aerosol is generated.

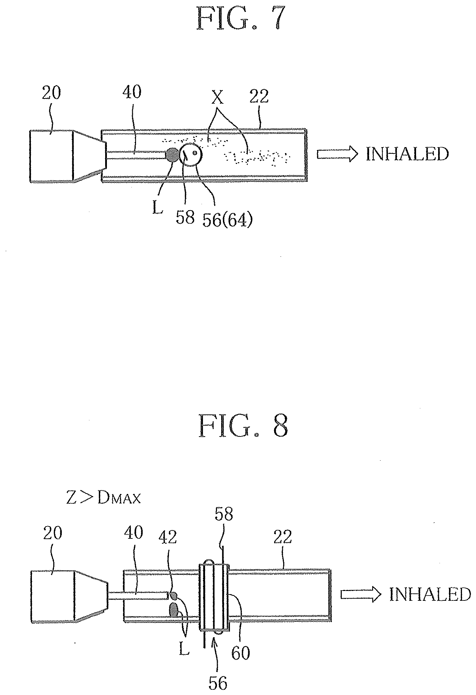

On the other hand, when the user inhales through the mouthpiece 14 of the aerosol inhalator in the state illustrated in FIG. 5, the solution is discharged from the discharge end 42 of the capillary tube 40 as mentioned above. Since the relationships indicated by the expressions (1) and (2) hold between the capillary tube 40 and the heater 56, the discharged solution L is reliably received on the outer surface of the heater 56, as shown in FIGS. 6 and 7. Where the net 64 is fitted around the outer surface of the heater 56, the discharged solution is received by the net 64 and then spreads over the net 64.

At this time, since the heating temperature of the outer surface of the heater 56 is already kept within the required temperature range, the discharged solution L is immediately atomized by being heated by the heater 56, generating an aerosol X inside the inner tube 22. The user can therefore inhale the aerosol X through the mouthpiece 14.

Also, where the heater 56 is provided with the net 64, the net 64 serves to enhance the wettability of the heater 56 with respect to the discharged solution L, so that the discharged solution L can be atomized over a wider area, enabling prompt generation of the aerosol.

As soon as the user ceases breathing in, the discharge of the solution from the discharge end 42 of the capillary tube 40 stops immediately. As is clear from the above explanation, since the gap Z between the discharge end 42 and the outer surface of the heater 56 is greater than at least the inner diameter $D_{CT}$ of the capillary tube 40, the solution in the discharge end 42 is not atomized by the radiant heat from the heater 56 insofar as the heating temperature of the outer surface of the heater 56 is kept within the aforementioned temperature range.

Accordingly, the generation of the aerosol stops at the same time as the cessation of the user's inhalation, so that the solution in the capillary tube 40 is not wasted.

As a result, the user can inhale the aerosol without fail each time he/she breathes in, and the amount of the aerosol inhaled by the user is determined by the intensity and duration of the user's inhalation.

On the other hand, even though the heating temperature of the outer surface of the heater 56 is kept within the required temperature range, the discharged solution L fails to be received on the outer surface of the heater 56 and drops to the inner surface of the inner tube 22, as shown in FIG. 8, if the relationship indicated by the aforementioned expression (2) is not fulfilled and the gap Z is greater than the maximum diameter $D_{MAX}$ of the liquid droplet of the solution. In such a case, the discharged solution L is not atomized, so that the user cannot inhale the aerosol.

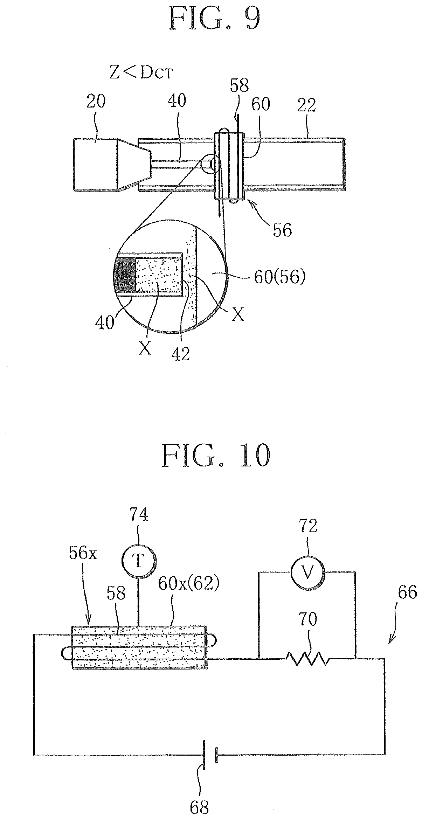

Conversely, if the gap Z is smaller than the inner diameter $D_{CT}$ of the capillary tube 40, the solution in the capillary tube 40 is possibly atomized by the radiant heat from the heater 56, as shown in FIG. 9. In this case, the aerosol X is generated independently of the user's inhalation, with the result that the solution in the liquid tank 20 is wasted.

Thus, in the case of the aerosol inhalator of this embodiment, the discharged solution L fails to be atomized, that is, the aerosol fails to be generated, or waste of the solution is unavoidable unless the relationships indicated by the expressions (1) and (2) are fulfilled and also the heating temperature of the outer surface of the heater 56 is kept within an appropriate temperature range.

Specifically, it is necessary that the relationships indicated by the expressions (1) and (2) be fulfilled and also that, where the solution is propylene glycol, the heating temperature of the outer surface of the heater 56 be kept within a temperature range of 180 to 280°C.

The aerosol inhalator of the embodiment does not include a control circuit for controlling the heat generated by the Nichrome wire 58. Thus, in order to keep the heating temperature of the outer surface of the heater 56 within the above temperature range, the thickness (volume) of the sheath element 60 needs to be properly set.

If the sheath element 60 has a larger thickness, it takes a longer time for heat to transfer from the Nichrome wire 58 to the outer surface of the heater 56 via the sheath element 60, and since the area of the outside surface of the sheath element 60 increases, the amount of heat radiated from the sheath element 60 also increases. Thus, it is thought that the larger the thickness of the sheath element 60, the lower the heating temperature of the outer surface of the heater 56 becomes.

In order to confirm such lowering of the heating temperature of the outer surface of the heater 56, the inventors hereof prepared heaters $56_A$ to $56_G$ which differed from one another only in the thickness of the sheath element 60. The sheath elements 60 of the heaters $56_A$ to $56_G$ had thicknesses progressively increasing in order of $56_A$ to $56_G$ each by a fixed increment.

FIG. 10 illustrates a heating test apparatus for a heater $56_X$ (X represents any one of A to G).

The heating test apparatus includes a power feed circuit 66 for applying a voltage to the heater $56_X$, and the power feed circuit 66 includes a direct-current power supply 68 capable of varying the applied voltage, a shunt resistor 70 (1 mΩ), and a voltmeter 72. The heater $56_X$ is connected in series with the shunt resistor 70.

Further, the heating test apparatus includes a temperature sensor 74, which is capable of measuring the temperature of the heater $56_X$, that is, the temperature of the outer surface of the sheath element 60. Specifically, the temperature sensor 74 includes a type K thermocouple.

When the heater $56_X$ is connected to the power feed circuit 66 as illustrated in FIG. 10, a voltage is applied from the direct-current power supply 68 to the Nichrome wire 58 of the heater $56_X$, so that the Nichrome wire 58 generates heat. The heat generated by the Nichrome wire 58 is transferred through the sheath element 60, thus increasing the temperature of the sheath element 60, and on the other hand is released to the outside from the outer surface of the sheath element 60.

Consequently, the heating temperature of the outer surface of the sheath element 60 is determined by a difference between the amount of heat generated by the Nichrome wire 58 and the amount of heat released from the sheath element 60, and the rate of temperature increase of the outer surface of the sheath element 60 is determined by the rate of heat transfer through the sheath element 60.

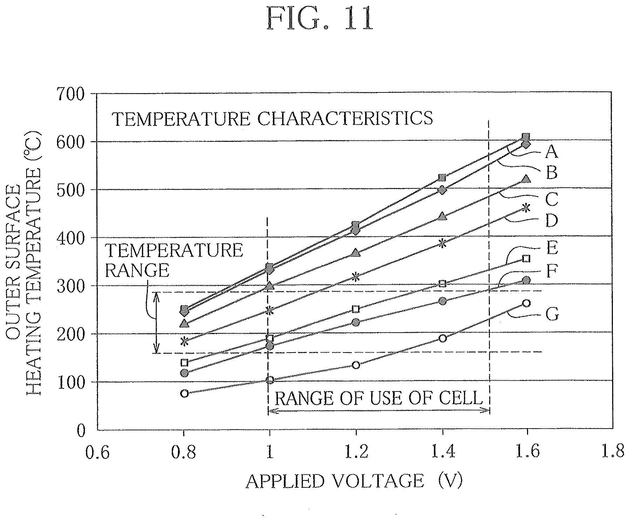

A heating test was conducted on the heater $56_X$ in such a manner that with the applied voltage, applied to the Nichrome wire 58 from the direct-current power supply 68, sequentially varied within a range of 0.8 V to 1.6 V, the heating temperature of the outer surface of the sheath element 60 was measured by the temperature sensor 74 with respect to each of the applied voltages of the Nichrome wire 58. The measurement results are shown in FIG. 11.

As is clear from FIG. 11, the outer surface of the sheath element 60 of the heater $56_X$ is heated to a higher temperature as the voltage applied to the Nichrome wire 58 increases.

Considering, however, ordinary use of the AA size cell 26 which is expected to apply a voltage of 1.0 V to 1.5 V, the heater $56_F$ alone is capable of keeping the heating temperature of the outer surface of the sheath element 60 within the aforementioned temperature range (180 to 280°C).

This means that where the heater $56_F$ is used as the heater 56 of the aerosol inhalator 10 of the embodiment, the heating temperature of the outer surface of the heater 56 can be kept within the required temperature range without the need to use a control circuit for controlling the voltage applied to the Nichrome wire 58.

Since the aerosol inhalator 10 need not be provided with such a control circuit, the load on the cell 26 is reduced, whereby the aerosol inhalator 10 can be used for a long period of time. Further, the use of the cell 26 serves to make the aerosol inhalator 10 smaller in size and slenderer, improving handiness of the aerosol inhalator 10.

If, on the other hand, the user inhales in a situation where the heating temperature of the outer surface of the heater 56 is lower than the aforementioned temperature range due to voltage reduction of the cell 26, the solution discharged from the capillary tube 40 may be insufficiently atomized and part of the discharged solution may possibly adhere to the inner surface of the inner tube 22.

Further, it is also conceivable that even though the heating temperature of the outer surface of the heater 56 is kept within the aforementioned temperature range, the generated aerosol condenses on the inner surface of the inner tube 22, with the result that the solution adheres to the inner surface of the inner tube 22.

In such cases, as the user inhales, the adherent solution may move toward the mouthpiece 14 and possibly flow into the user's mouth.

However, since the absorbent sleeve 48, which is a paper tube or paper filter, is arranged between the inner tube 22 and the mouthpiece 14, the adherent solution, if moved toward the mouthpiece 14, is reliably absorbed into the absorbent sleeve 48 and does not flow into the user's mouth.

The present invention is not limited to the aerosol inhalator 10 of the foregoing embodiment and may be modified in various ways.

As regards the heater 56, for example, the resistance heating element is not limited to Nichrome wire, and the cross-sectional shape of the heater 56 is not limited to circle and may instead be ellipse, polygon or the like.

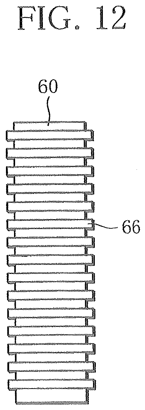

The sheath element 60 may be made of metal and, as shown in FIG. 12 by way of example, may have a rough outer surface 66 formed on at least a portion thereof where to receive the discharged solution, in place of the aforementioned net 64. The rough outer surface 66 is constituted, for example, by a large number of narrow annular grooves spaced from each other in the axial direction of the sheath element 60, and when the discharged solution is received on the outer surface 66 of the sheath element 60, the annular grooves serve to spread the discharged solution, like the net 64.

Further, where the sheath element 60 of the heater 56 and the inner tube 22 are to be made of the same ceramic, the sheath element 60 and the inner tube 22 are preferably formed as a one-piece molded article, and in this case, the number of parts of the aerosol inhalator can be reduced.

**Reference Signs List**

12: outer tube
14: mouthpiece
18: power supply unit
20: liquid tank
22: inner tube
26: cell
40: capillary tube
42: discharge end
48: absorbent sleeve (paper tube, paper filter)
50: atmospheric port
52: atmosphere-exposed opening
56: heater
58: Nichrome wire (resistance heating element)
60: sheath element
64: net (wetting enhancement element)

**Claims**

1. An aerosol inhalator comprising:

a suction path (50, 22) connecting an atmosphere-exposed opening (50) and a mouthpiece (14) to each other and permitting air to flow from the atmosphere-exposed opening (50) toward the mouthpiece (14);
a solution supply device (20, 40) configured to supply a solution (L) from which an aerosol is to be generated, said solution supply device including

a solution supply source (20) storing the solution (L), and
a capillary tube (40) connected to the solution supply source (20) and having a discharge end (42) located in said suction path and opening in a direction toward the mouthpiece (14), the capillary tube (40) guiding the solution (L) from the solution supply source to the discharge end (42) and, when the flow of air is produced within said suction path, allowing the solution (L) to be discharged from the discharge end (42); and

a heater device (26, 56) configured to receive the solution (L) discharged from the discharge end (42) of the capillary tube (40) and atomize the received solution (L) by heating, said heater device including

a power supply (26), and
an electric heater (56) arranged immediately downstream of the discharge end (42) and facing the discharge end (42) while permitting the flow of air, the heater (56) being configured to generate heat when applied with a voltage from the power supply (26), wherein the heater (56) extends in a direction perpendicular to an axis of said suction path and traverses said suction path;
wherein the discharge end (42) of the capillary tube (40) is arranged in a position such that the discharge end (42) is hidden by the heater (56) when an interior of said suction path is viewed from the mouthpiece side,

wherein a distance (Z) is secured between the discharge end (42) of the capillary tube (40) and the heater (56), **characterized in that** the distance (Z) is adapted so that a liquid droplet of the solution (L) is formed at the discharge end (42) by surface tension of the solution (L) and the formed liquid droplet is directly received by a surface of the electric heater (56).

2. The aerosol inhalator according to claim 1, wherein the capillary tube (40) extends coaxially with said suction path.

3. The aerosol inhalator according to claim 1, wherein the distance (Z) is longer than an inner diameter ($D_{CT}$) of the capillary tube (40).

4. The aerosol inhalator according to claim 1, wherein the heater (56) has a cylindrical shape and an outer diameter ($D_o$), and the capillary tube (40) has an inner diameter smaller than the outer diameter ($D_o$) of the heater (56).

5. The aerosol inhalator according to claim 1, wherein the heater (56) has a non-smooth region (64) on at least part of an outer surface thereof and receives the discharged solution (L) on the non-smooth region (64).

6. The aerosol inhalator according to claim 1, wherein the heater (56) includes a resistance heating element (58), and a sheath element (60) enclosing the resistance heating element (58), the sheath element (60) cooperating with the heating element (58) to keep heating temperature of an outer surface of the heater (56) within a predetermined temperature range required to atomize the discharged solution (L).

7. The aerosol inhalator according to claim 6, wherein the heater (56) further includes a wetting enhancement element (64) configured to cause the discharged solution (L) received on an outer surface of the heater (56) to spread over the outer surface.

8. The aerosol inhalator according to claim 1, wherein the power supply includes a commercially available battery cell (26).

**Patentansprüche**

1. Aerosolinhalator, umfassend:

eine Saugstrecke (50, 22), die eine zur Umgebung hin offene Öffnung (50) und ein Mundstück (14) miteinander verbindet und ermöglicht, dass Luft von der zur Umgebung hin offenen Öffnung (50) in Richtung des Mundstücks (14) strömt;

eine Lösungszufuhrvorrichtung (20, 40), die so ausgelegt ist, dass sie eine Lösung (L) zuführt, aus der ein Aerosol erzeugt werden soll, wobei die Lösungszufuhrvorrichtung Folgendes aufweist:

eine Lösungsversorgungsquelle (20), welche die Lösung (L) bevorratet, und

ein Kapillarrohr (40), das mit der Lösungsversorgungsquelle (20) verbunden ist und ein Abgabeende (42) aufweist, das sich in der Saugstrecke befindet und in eine Richtung zum Mundstück (14) hin öffnet, wobei das Kapillarrohr (40) die Lösung (L) aus der Lösungsversorgungsquelle zum Abgabeende (42) leitet und, wenn der Luftstrom in der Saugstrecke erzeugt wird, ermöglicht, dass die Lösung (L) aus dem Abgabeende (42) abgegeben wird; und

eine Heizvorrichtung (26, 56), die so ausgelegt ist, dass sie die Lösung (L) aufnimmt, die aus dem Abgabeende (42) des Kapillarrohrs (40) abgegeben wird, und die aufgenommene Lösung (L) durch Erwärmen zerstäubt, wobei die Heizvorrichtung Folgendes aufweist:

eine Stromversorgung (26) und

ein elektrisches Heizelement (56), das in Strömungsrichtung unmittelbar hinter dem Abgabeende (42) angeordnet und dem Abgabeende (42) zugewandt ist, während es gleichzeitig den Luftstrom zulässt, wobei das Heizelement (56) so ausgelegt ist, dass es Wärme erzeugt, wenn eine Spannung von der Stromversorgung (26) angelegt wird, wobei das Heizelement (56) in einer Richtung senkrecht zu einer Achse der Saugstrecke verläuft und quer durch die Saugstrecke verläuft;

wobei das Abgabeende (42) des Kapillarrohrs (40) derart in einer Position angeordnet ist, dass das Abgabeende (42) durch das Heizelement (56) verdeckt wird, wenn ein Innenraums der Saugstrecke von der Seite des Mundstücks aus betrachtet wird,

wobei ein Abstand (Z) zwischen dem Abgabeende (42) des Kapillarrohrs (40) und dem Heizelement (56) gewährleistet ist, **dadurch gekennzeichnet, dass**

der Abstand (Z) so ausgelegt ist, dass ein Flüssigkeitströpfchen der Lösung (L) durch Oberflächenspannung der Lösung (L) am Abgabeende (42) entsteht und das entstandene Flüssigkeitströpfchen direkt von einer Fläche des elektrischen Heizelements (56) aufgenommen wird.

2. Aerosolinhalator nach Anspruch 1, wobei das Kapillarrohr (40) koaxial mit der Saugstrecke verläuft.

3. Aerosolinhalator nach Anspruch 1, wobei der Abstand (Z) länger ist als ein Innendurchmesser ($D_{CT}$) der Kapillarröhre (40).

4. Aerosolinhalator nach Anspruch 1, wobei das Heizelement (56) eine zylindrische Form und einen Außendurchmesser ($D_O$) aufweist und das Kapillarrohr (40) einen Innendurchmesser aufweist, der kleiner ist als der Außendurchmesser ($D_O$) des Heizelements (56).

5. Aerosolinhalator nach Anspruch 1, wobei das Heizelement (56) einen nicht glatten Bereich (64) auf zumindest einem Teil einer Außenfläche davon aufweist und die abgegebene Lösung (L) an dem nicht glatten Bereich (64) aufnimmt.

6. Aerosolinhalator nach Anspruch 1, wobei das Heizelement (56) ein Widerstandsheizelement (58) sowie ein Hüllenelement (60) aufweist, welches das Widerstandsheizelement (58) umgibt, wobei das Hüllenelement (60) mit dem Heizelement (58) so zusammenwirkt, dass die Heiztemperatur einer Außenfläche des Heizelements (56) innerhalb eines vorgegebenen Temperaturbereichs bleibt, die zum Zerstäuben der abgegebenen Lösung (L) notwendig ist.

7. Aerosolinhalator nach Anspruch 6, wobei das Heizelement (56) ferner ein Befeuchtungsverbesserungselement (64) aufweist, das so ausgelegt ist, dass es bewirkt, dass sich die abgegebene Lösung (L), die an einer Außenfläche des Heizelements (56) aufgenommen ist, über der Außenfläche verteilt.

8. Aerosolinhalator nach Anspruch 1, wobei die Stromversorgung eine handelsübliche Batteriezelle (26) aufweist.

**Revendications**

1. Inhalateur d'aérosol comprenant :

un chemin d'aspiration (50, 22) reliant une ouverture exposée à l'atmosphère (50) et un embout buccal (14) l'un à l'autre et permettant à l'air de circuler à partir de l'ouverture exposée à l'atmosphère (50) vers l'embout buccal (14) ;

un dispositif d'alimentation en solution (20, 40) configuré pour fournir une solution (L) à partir de laquelle un aérosol doit être généré, ledit dispositif d'alimentation en solution comprenant

une source d'alimentation en solution (20) stockant la solution (L) et

un tube capillaire (40) relié à la source d'alimentation en solution (20) et ayant une extrémité de décharge (42) située dans ledit chemin d'aspiration et s'ouvrant dans une direction vers l'embout buccal (14), le tube capillaire (40) guidant la solution (L) à partir de la source d'alimentation en solution vers l'extrémité de décharge (42) et, lorsque la circulation d'air est produite dans ledit chemin d'aspiration, permettant à la solution (L) d'être déchargée à partir de l'extrémité de décharge (42) ; et

un dispositif de chauffage (26, 56) configuré pour recevoir la solution (L) déchargée à partir de l'extrémité de décharge (42) du tube capillaire (40) et atomiser la solution reçue (L) par chauffage, ledit dispositif de chauffage comprenant

une alimentation électrique (26), et

un réchauffeur électrique (56) agencé immédiatement en aval de l'extrémité de décharge (42) et faisant face à l'extrémité de décharge (42) tout en permettant la circulation d'air, le réchauffeur (56) étant configuré pour générer de la chaleur lorsqu'il se voit appliquer une tension à partir de l'alimentation électrique (26), dans lequel le réchauffeur (56) s'étend dans une direction perpendiculaire à un axe dudit chemin d'aspiration et traverse ledit chemin d'aspiration ;

dans lequel l'extrémité de décharge (42) du tube capillaire (40) est agencée dans une position telle que l'extrémité de décharge (42) est cachée par le réchauffeur (56) lorsqu'un intérieur dudit trajet d'aspiration est vu du côté de l'embout buccal,

dans lequel une distance (Z) est fixée entre l'extrémité de décharge (42) du tube capillaire (40) et le réchauffeur (56), **caractérisé en ce que**

la distance (Z) est conçue de telle sorte qu'une gouttelette de liquide de la solution (L) est formée au niveau de l'extrémité de décharge (42) par la tension superficielle de la solution (L), et la gouttelette de liquide formée est directement reçue par une surface du réchauffeur électrique (56).

2. Inhalateur d'aérosol selon la revendication 1, dans lequel le tube capillaire (40) s'étend coaxialement audit chemin d'aspiration.

3. Inhalateur d'aérosol selon la revendication 1, dans lequel la distance (Z) est supérieure à un diamètre intérieur ($D_{CT}$) du tube capillaire (40).

4. Inhalateur d'aérosol selon la revendication 1, dans lequel le réchauffeur (56) a une forme cylindrique et un diamètre extérieur ($D_o$), et le tube capillaire (40) a un diamètre intérieur inférieur au diamètre extérieur ($D_o$) du réchauffeur (56).

5. Inhalateur aérosol selon la revendication 1, dans lequel le réchauffeur (56) comporte une région non lisse (64) sur au moins une partie de sa surface extérieure, et reçoit la solution déchargée (L) sur la région non lisse (64).

6. Inhalateur d'aérosol selon la revendication 1, dans lequel le réchauffeur (56) comprend un élément de chauffage à résistance (58), et un élément de gaine (60) enfermant l'élément de chauffage à résistance (58), l'élément de gaine (60) coopérant avec l'élément de chauffage (58) pour maintenir la température de chauffage d'une surface extérieure du réchauffeur (56) dans une plage de températures prédéterminée requise pour atomiser la solution déchargée (L).

7. Inhalateur d'aérosol selon la revendication 6, dans lequel le réchauffeur (56) comprend en outre un élément d'amélioration de mouillage (64) configuré pour amener la solution déchargée (L) reçue sur une surface extérieure du réchauffeur (56) à se répandre sur la surface extérieure.

8. Inhalateur d'aérosol selon la revendication 1, dans lequel l'alimentation électrique comprend une cellule de batterie disponible dans le commerce (26).

# FIG. 1

# FIG. 2

# FIG. 3

# FIG. 4

# FIG. 5

# FIG. 6

# FIG. 7

# FIG. 8

# FIG. 9

$Z < D_{CT}$

# FIG. 10

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008105918A1 PCT
- JP 2006524494 A
- WO 9748293 A

- JP H11199989551 B
- WO 9501137 A1
- EP 0845220 A1